(19)

Europäisches Patentamt
European Patent Office
Office européen des brevets

(11) **EP 4 664 154 A1**

(12) **EUROPEAN PATENT APPLICATION**

(43) Date of publication:
**17.12.2025 Bulletin 2025/51**

(21) Application number: **24182886.2**

(22) Date of filing: **18.06.2024**

(51) International Patent Classification (IPC):
*G01S 7/52* (2006.01)     *G01S 15/89* (2006.01)
*A61B 8/08* (2006.01)

(52) Cooperative Patent Classification (CPC):
**G01S 7/52049; A61B 8/0883; A61B 8/5207;
A61B 8/5269; G01S 7/52095; G01S 15/8915;
G01S 15/8997**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **11.06.2024  US 202463658497 P**

(71) Applicant: **Koninklijke Philips N.V.
5656 AG Eindhoven (NL)**

(72) Inventor: **ROBERT, Jean-Luc Francois-Marie
Eindhoven (NL)**

(74) Representative: **Philips Intellectual Property &
Standards
High Tech Campus 52
5656 AG Eindhoven (NL)**

(54) **METHOD AND SYSTEM FOR CORRECTING ULTRASOUND IMAGING DATA**

(57)    A system and method for correcting ultrasound imaging data. Ultrasound imaging data is received from a beamforming unit and processed to estimate a delay error profile. The delay error profile is backpropagated to determine weights and delays of a transmit beam used to acquire the ultrasound imaging data. The ultrasound imaging data is processed using a transmit compounding algorithm, using the determined weights and delays as coefficients of the transmit compounding algorithm.

700

| Receive ultrasound imaging data | 710 |

| Estimate delay error | 720 |

| Determine weights and delays | 730 |

| Generate corrected ultrasound imaging data | 740 |

FIG. 7

**Description**

FIELD OF THE INVENTION

The invention relates to the field of ultrasound imaging.

BACKGROUND OF THE INVENTION

One of the key difficulties in ultrasound imaging is speed of sound variation in an area being imaged. This causes aberration in both transmitted and received ultrasound beams, reducing image quality. The main source of speed of sound variation is the presence of fat in the superficial layers or the liver. The speed of sound in fat is considerably lower than in other tissue.

There is an ongoing desire to reduce the effects of speed of sound variation in ultrasound images.

SUMMARY OF THE INVENTION

The invention is defined by the claims.

According to examples in accordance with an aspect of the invention, there is provided a computer-implemented method for correcting ultrasound imaging data, the computer-implemented method comprising: receiving, from an ultrasound beamforming unit, ultrasound imaging data for a subject; processing the ultrasound imaging data to estimate a delay error for each channel of the ultrasound beamforming unit; backpropagating the estimated delay error for each channel to determine weights and delays for a transmit beam used to acquire the ultrasound imaging data; and processing the ultrasound imaging data using a transmit compounding algorithm to generate corrected ultrasound imaging data, wherein the determined weights and delays are used as coefficients of the transmit compounding algorithm.

In this way, transmit aberration may be corrected retrospectively, thus reducing the effects of speed of sound variation in the ultrasound imaging data, and improving resolution and sharpness of the ultrasound imaging data.

Retrospective correction of these effects is advantageous, as this does not require modification of the transmit aperture, which necessitates testing to check that the modified transmit aperture meets safety requirements, or changes to transmit delays, which is cumbersome.

In some examples, the computer-implemented method further comprises using the estimated delay error for each channel to correct receive aberration in the ultrasound imaging data.

Correcting both transmit aberration and receive aberration in the ultrasound imaging data provides improved image quality compared with correcting only one or the other.

In some examples, the weights and delays for the transmit beam are determined by backpropagating the estimated delay error for each channel using a simulation of the transmit beam.

In some examples, the weights and delays for the transmit beam are determined by backpropagating the estimated delay error for each channel using an analytical formula approximating the transmit beam.

In some examples, the analytical formula is a Rayleigh-Sommerfeld formula. The use of a Rayleigh-Sommerfeld formula allows the transmit beam to be approximated to a high level of precision.

In some examples, the weights and delays for the transmit beam are determined for each line of ultrasound imaging data.

This allows local aberrations to be corrected, further improving the quality of the ultrasound imaging data.

In some examples, the weights and delays for the transmit beam are determined for each of a plurality of depths.

By making the correction to the ultrasound imaging data depth-dependent, the quality of the ultrasound imaging data is further improved.

In some examples, the delay error for each channel is estimated using a cross-correlation technique.

Cross-correlation methods are less computationally intensive than other techniques for estimating the delay error.

In some examples, the ultrasound imaging data is cardiac ultrasound imaging data.

Transmit aberration correction is particularly advantageous in cardiac ultrasound applications, as the transmit and receive aperture are similarly sized in cardiac ultrasound. Transmit aberration therefore provides a greater contribution to the overall error in cardiac ultrasound imaging compared to other ultrasound applications in which the receive aperture is significantly larger than the transmit aperture.

There is also provided a computer program product comprising computer program code means which, when executed on a computing device having a processing system, cause the processing system to perform all of the steps of any of the methods described above.

According to examples in accordance with another aspect of the invention, there is provided a processing system for correcting ultrasound imaging data, the processing system being configured to: receive, from an ultrasound beamforming unit, ultrasound imaging data for a subject; process the ultrasound imaging data to estimate a delay error for each channel of the ultrasound beamforming unit; backpropagate the estimated delay error for each channel to determine weights and delays for a transmit beam used to acquire the ultrasound imaging data; and process the ultrasound imaging data using a transmit compounding algorithm to generate corrected ultrasound imaging data, wherein the determined weights and delays are used as coefficients of the transmit compounding algorithm.

In some examples, the processing system is further configured to use the estimated delay error for each channel to correct receive aberration in the ultrasound imaging data.

In some examples, the weights and delays for the transmit beam are determined for each line of ultrasound imaging data.

In some examples, the weights and delays for of the transmit beam are determined for each of a plurality of depths.

There is also provided an ultrasound system, comprising: the processing system described above; and an ultrasound beamforming unit configured to acquire ultrasound imaging data.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates an exemplary ultrasound system;
Fig. 2 illustrates an ultrasound system, according to an embodiment of the invention;
Fig. 3 illustrates an error delay profile for example ultrasound imaging data;
Fig. 4 illustrates a simulation 400 of the aberrated transmit beam used to acquire the example ultrasound imaging data from which the error delay profile shown in Figure 3 was estimated;
Fig. 5 illustrates a simulation of the transmit beam that does not take speed of sound errors into account;
Fig. 6 illustrates three ultrasound images generated using the same example ultrasound imaging data; and
Fig. 7 illustrates a computer-implemented method for correcting ultrasound imaging data, according to an embodiment of the invention.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for correcting ultrasound imaging data. Ultrasound imaging data is received from a beamforming unit and processed to estimate a delay error profile. The delay error profile is backpropagated to determine weights and delays of a transmit beam used to acquire the ultrasound imaging data. The ultrasound imaging data is processed using a transmit compounding algorithm, using the determined weights and delays as coefficients of the transmit compounding algorithm.

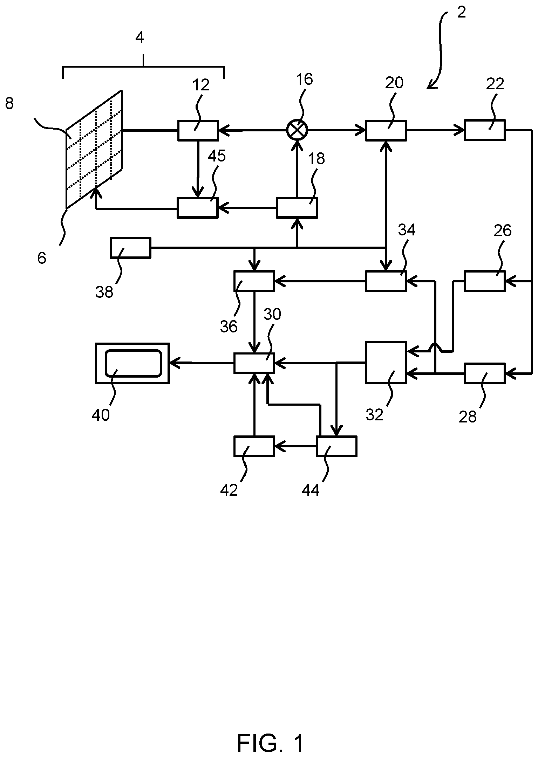

The general operation of an exemplary ultrasound system will first be described, with reference to Fig. 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 1 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

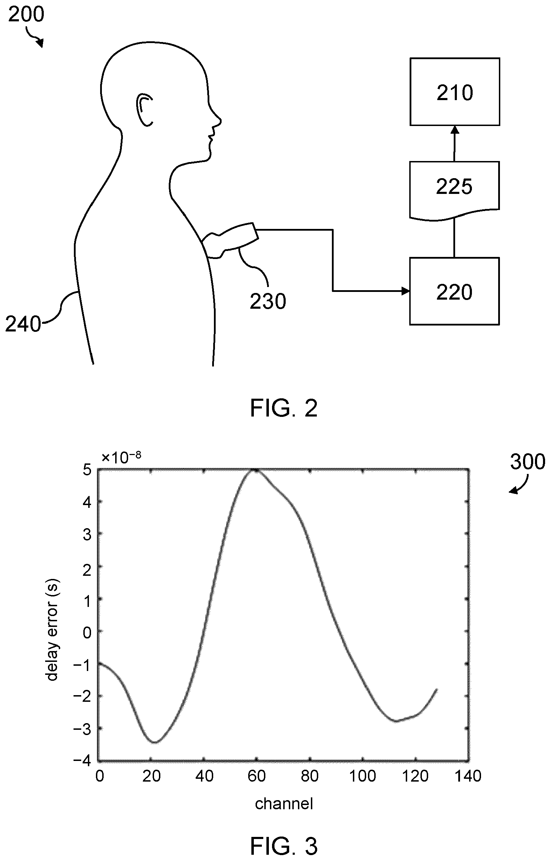

Fig. 2 illustrates an ultrasound system 200, according to an embodiment of the invention. The system comprises a processing system 210 for correcting ultrasound imaging data, and an ultrasound beamforming unit 220. The processing system 210 is, itself, an embodiment of the invention.

The ultrasound beamforming unit 220 is connected to an ultrasound transducer probe 230, and is configured to acquire ultrasound imaging data 225 of a subject 240 using the ultrasound transducer probe. The transducer probe may be a conventional ultrasound transducer probe, as described above with reference to Fig. 1. The ultrasound beamforming unit may be any multiline ultrasound beamforming unit that is capable of performing an estimation of receive aberration (e.g. through cross-correlation).

The ultrasound imaging data 225 may be acquired using any shape of transmit beam (e.g. a focused beam, a diverging beam, a plane wave, etc.). All shapes of transmit beam are affected by aberration caused by speed of sound variation. In some examples, the ultrasound imaging data may be cardiac ultrasound imaging data, for which transmit aberration has a larger effect due to the transmit aperture size; however, the invention is not limited to cardiac ultrasound imaging data, and may be used to correct any ultrasound imaging data in which speed of sound errors are present.

The processing system 210 is configured to receive the ultrasound imaging data 225 from the ultrasound beamforming unit 220, and to process the ultrasound imaging data to estimate a delay error for each channel of the ultrasound beamforming unit. The delay error may be estimated in the time domain or the frequency domain.

Any suitable technique may be used to estimate the delay error for each channel of the ultrasound beamforming unit. In some examples, a cross-correlation technique may be used. For instance, the ultrasound signal received by each channel may be cross-correlated (in time or frequency domain) with the ultrasound signal received by a neighboring channel to determine the difference in time arrival or phase between the channels. The differences between neighboring pair of channels may be averaged to reduce variance. Alternatively, the ultrasound signal received by each channel may be cross-correlated with beamsum (the sum of the ultrasound signals across all channels). Suitable cross-correlation techniques for estimating the delay error are, for example, described in Karaman et al. (1993), "A phase aberration correction method for ultrasound imaging", IEEE Transactions on Ultrasonics, Ferroelectrics and Frequency Control, 40(4):275-282 and in US Patent No. 5,388,461.

In other examples, the delay error for each channel may be estimated by determining the singular value decomposition of a covariance matrix of the ultrasound signals, as described, for instance, in Robert and Fink (2008), "Green's function estimation in speckle using the decomposition of the time reversal operator: Application to aberration correction in medical imaging", J. Acoust. Soc. Am. 123(2):866-877, and Måsøy et al. (2007), "Variance analysis of arrival time and amplitude estimates from random speckle signal", J. Acoust. Soc. Am. 121(1):286-297. Further examples of suitable techniques for estimating the delay error for each channel will be apparent to the skilled person.

Fig. 3 illustrates an error delay profile 300 for example ultrasound imaging data. The y-axis represents the delay error in seconds, while the x-axis represents the channel.

Returning to Fig. 2, having estimated the delay error for each channel of the ultrasound beamforming unit, the processing system 210 is configured to backpropagate the estimated delay error for each channel to determine weights and delays for the (aberrated) transmit beam used to acquire the ultrasound imaging data 225. In this way, the determined weights and delays take aberration in the transmit beam into account. In some examples, the weights and delays may be determined for each line of the ultrasound imaging data and/or for each of a plurality of depths.

The weights and delays determined may depend on the transmit compound algorithm to be used to generate corrected ultrasound data (see below). For example, the weights and delays may comprise at least an amplitude and phase delay of the transmit beam.

For instance, the weights for the transmit beam may comprise an amplitude of the transmit beam at each of a plurality of locations $(x, z)$ in the imaging field, where x is the azimuthal position (with x = 0 corresponding to the center axis of the transmit beam) and z is the depth:

$$\text{weight}\,(x, z) = \text{amplitude}\,(x, z) \tag{1}$$

The delays for the transmit beam may comprise a difference between a propagation time taken for the transmit wavefront to reach each of a plurality of locations $(x, z)$ in the imaging field and a propagation time to reach a point on the center axis (x = 0) at the same depth z as the location $(x, z)$:

$$\text{delay}\,(x, z) = \text{propagation time}\,(x, z) \,-\, \text{propagation time}\,(0, z) \tag{2}$$

where the propagation time is determined based on the phase delay.

In some examples, the weights and delays for the transmit beam used to acquire the ultrasound imaging data 225 may be determined by backpropagating the estimated delay error for each channel using a simulation of the transmit beam (e.g. a finite element simulation, a non-linear simulation, etc.). For instance, a simulation of the transmit beam that does not take speed of sound errors into account may first be generated, based on the focal depth and aperture size. The simulation may then be modified until the simulation of the transmit beam corresponds to the estimated delay error for each channel. In some examples, the simulation may be further modified to correct the amplitude of the simulation of the transmit beam (e.g. based on an amplitude of the cross-correlation function). The weights and delays for the transmit beam may then be determined based on the modified transmit beam.

In other examples, the weights and delays for the transmit beam used to acquire the ultrasound imaging data 225 may be determined by backpropagating the estimated delay error for each channel using an analytical formula that approximates the transmit beam (i.e. that models at least the amplitude and phase of the transmit beam pattern). An analytical formula that approximates the transmit beam without taking speed of sound errors into account may first be defined; the coefficients of the analytical formula may then be modified to account for the estimated delay error for each channel. The coefficients may further be modified to adjust the amplitude (e.g. based on the amplitude of the cross-correlation function). The weights and delays for the transmit beam may be determined based on the modified coefficients.

Any suitable analytical formula may be used to backpropagate the estimated delay error. In some examples, the analytical formula may be a formula that models and sums the Green's function or radiation pattern of transducer array elements, such as the Rayleigh-Sommerfeld formula. In such a case, the delay applied to each element may include the delay of the non-aberrated transmit beam plus the estimated delay error for the element. In some examples, the analytical formula may use an approximation such as the Fraunhoffer approximation or Fresnel approximation, and a weight may be applied to each element (e.g. a weight proportional to the amplitude of the cross-correlation function, a weight corresponding to a desired apodization function, or a weight determined to compensate for an estimated attenuation).

In some examples, the backpropagation of the estimated delay error may be performed in the angular spectrum domain, by performing a spatial Fourier transform on a complex aperture function for which the phase corresponds to the estimated delay error for each channel. The signal in the angular spectrum domain may then be backpropagated to the imaging depth (e.g. to the imaging depth for each pixel of the ultrasound imaging data) by multiplying by a corresponding transfer function, before performing an inverse spatial Fourier transform. The definition of a suitable transfer function (e.g. a complex exponential) will be readily apparent to the skilled person.

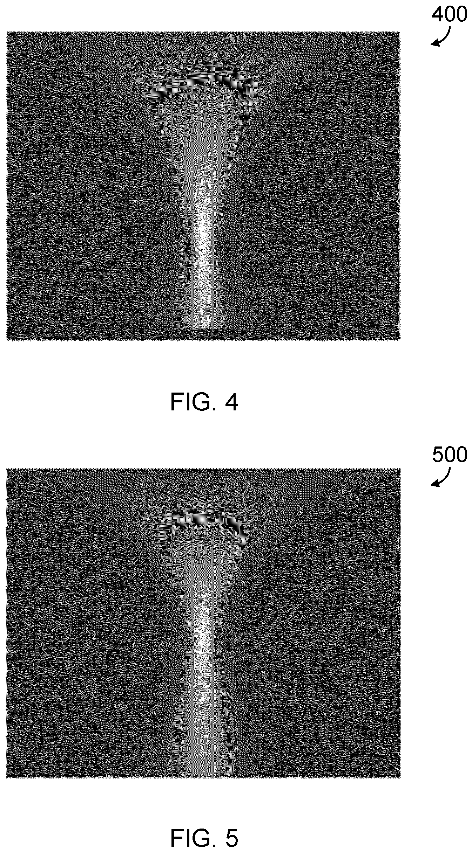

Fig. 4 illustrates a simulation 400 of the (aberrated) transmit beam used to acquire the example ultrasound imaging data from which the error delay profile 300 shown in Fig. 3 was estimated. The simulation 400 of the transmit beam was generated by backpropagating the per-channel error delay.

For comparison, Fig. 5 illustrates a simulation 500 of the transmit beam that does not take speed of sound errors into account. As a comparison of Figs. 4 and 5 shows, the speed of sound error results in a shift in the focal depth of the beam and a widening of the beam at the focal depth.

Returning to Fig. 2, the processing system 210 is configured to process the ultrasound imaging data 225 using a transmit compounding algorithm, using the determined weights and delays as coefficients of the transmit compounding algorithm, to generate corrected ultrasound imaging data. The transmit compounding algorithm may be any algorithm that combines weights and delays to reconstruct a transmit beam (e.g. a transmit beam reconstruction, XBR, algorithm). For instance, a suitable transmit compounding algorithm is described in US Patent No. 8,137,272 B2.

Transmit compounding algorithms are generally used to retrospectively refocus a transmit beam at depths other than the focal depth. The inventor has recognized that a transmit compounding algorithm may further be modified to correct for a lack of focus due to speed of sound errors, by determining the weights and delays of the aberrated transmit beam and using these weights and delays in the transmit compounding algorithm.

In some examples, the processing system 210 is further configured to correct receive aberration in the ultrasound imaging data using the estimated delay error for each channel of the ultrasound beamforming unit (i.e. by applying the estimated delay errors as corrections to the ultrasound imaging data). Any suitable technique may be used to correct receive aberration in the ultrasound imaging data; see, for example, Måsøy et al. (2022), "Aberration correction in 2D echocardiography", IEEE Transactions on Medical Imaging, DOI: 10.36227/techrxiv.19739905.v1, and Ali et al. (2023), "Aberration correction in diagnostic ultrasound: A review of the prior field and current directions", Zeitschrift für Medizinische Physik, 33(3):267-291.

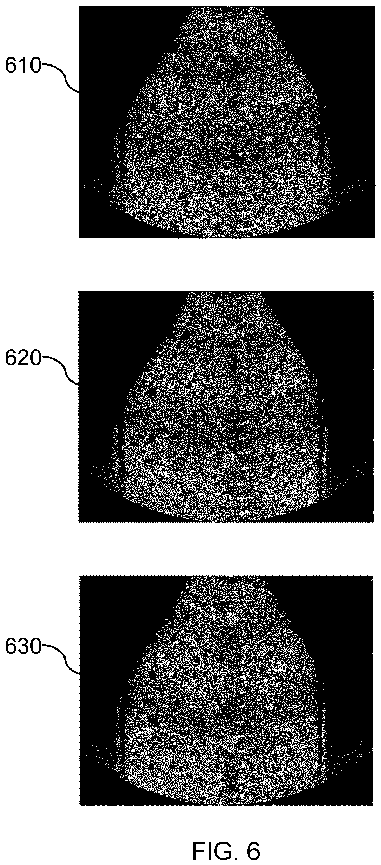

Fig. 6 illustrates three ultrasound images generated using the same example ultrasound imaging data, acquired using a Philips C5-1 ultrasound transducer. In image 610, no correction has been applied to the ultrasound imaging data. In image 620, receive aberration has been corrected. Image 620 shows an improvement in image quality compared with image 610. In image 630, transmit aberration has been corrected, using the approach described above, in addition to correcting receive aberration. Image 630 shows a further improvement in image quality. The proposed approach for correcting transmit aberration would have a still greater effect on image quality with ultrasound imaging data acquired using a larger transmit aperture (as a larger transmit aperture results in greater aberration in the transmit beam).

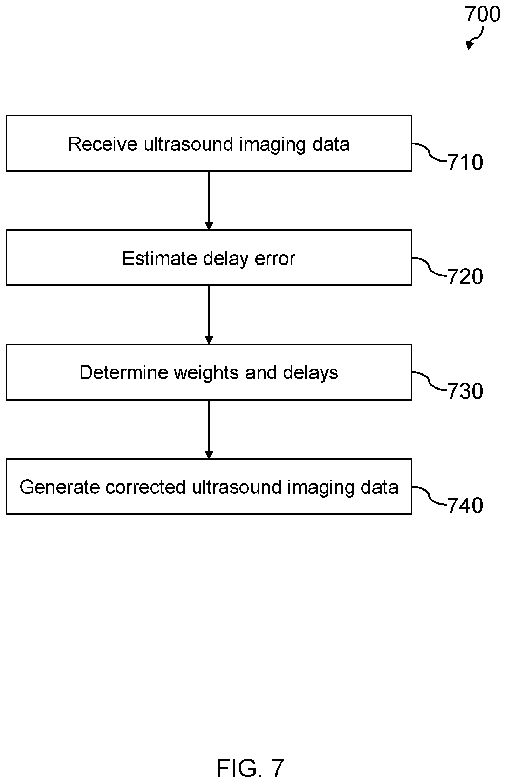

Fig. 7 illustrates a computer-implemented method 700 for correcting ultrasound imaging data, according to an embodiment of the invention.

The method 700 begins at step 710, at which ultrasound imaging data for a subject is received from an ultrasound beamforming unit.

At step 720, the ultrasound imaging data is processed to estimate a delay error for each channel of the ultrasound beamforming unit. Any of the methods described above may be used to estimate the delay error. For instance, a cross-correlation technique may be used.

At step 730, the estimated delay error for each channel is backpropagated to determine weights and delays of a transmit beam used to acquire the ultrasound imaging data. The weights and delays may, for example, be determined by backpropagating the estimated delay error for each channel using a simulation of the transmit beam or an analytical formula approximating the transmit beam (e.g. a Rayleigh-Sommerfeld formula), as described above. In some examples, the weights and delays may be determined for each line of the ultrasound imaging data, and/or for each of a plurality of depths.

At step 740, the ultrasound imaging data is processed using a transmit compounding algorithm to generate corrected ultrasound imaging data. The determined weights and delays are used as coefficients of the transmit compounding algorithm.

In some examples, the method 700 may further comprise a step (not shown in Fig. 7) of using the estimated delay error for each channel to correct receive aberration in the ultrasound imaging data.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing system may be implemented by a single processing system or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A computer-implemented method (700) for correcting ultrasound imaging data, the computer-implemented method comprising:

   receiving, from an ultrasound beamforming unit (220), ultrasound imaging data (225) for a subject (240);
   processing the ultrasound imaging data to estimate a delay error for each channel of the ultrasound beamforming unit;
   backpropagating the estimated delay error for each channel to determine weights and delays for a transmit beam used to acquire the ultrasound imaging data; and
   processing the ultrasound imaging data using a transmit compounding algorithm to generate corrected ultrasound imaging data, wherein the determined weights and delays are used as coefficients of the transmit compounding algorithm.

2. The computer-implemented method (700) of claim 1, further comprising using the estimated delay error for each channel to correct receive aberration in the ultrasound imaging data (225).

3. The computer-implemented method (700) of claim 1 or 2, wherein the weights and delays for the transmit beam are determined by backpropagating the estimated delay error for each channel using a simulation of the transmit beam.

4. The computer-implemented method (700) of claim 1 or 2, wherein the weights and delays for the transmit beam are determined by backpropagating the estimated delay error for each channel using an analytical formula approximating the transmit beam.

5. The computer-implemented method (700) of claim 4, wherein the analytical formula is a Rayleigh-Sommerfeld formula.

6. The computer-implemented method (700) of any of claims 1 to 5, wherein the weights and delays for the transmit beam are determined for each line of the ultrasound imaging data (225).

7. The computer-implemented method (700) of any of claims 1 to 6, wherein the weights and delays for the transmit beam are determined for each of a plurality of depths.

8. The computer-implemented method (700) of any of claims 1 to 7, wherein the delay error for each channel is estimated using a cross-correlation technique.

9. The computer-implemented method (700) of any of claims 1 to 8, wherein the ultrasound imaging data (225) is cardiac ultrasound imaging data.

10. A computer program product comprising computer program code means which, when executed on a computing device having a processing system, cause the processing system to perform all of the steps of the method (700) according to any of claims 1 to 9.

11. A processing system (210) for correcting ultrasound imaging data, the processing system being configured to:

receive, from an ultrasound beamforming unit (220), ultrasound imaging data (225) for a subject (240);
process the ultrasound imaging data to estimate a delay error for each channel of the ultrasound beamforming unit;
backpropagate the estimated delay error for each channel to determine weights and delays for a transmit beam used to acquire the ultrasound imaging data; and
process the ultrasound imaging data using a transmit compounding algorithm to generate corrected ultrasound imaging data, wherein the determined weights and delays are used as coefficients of the transmit compounding algorithm.

12. The processing system (210) of claim 11, wherein the processing system is further configured to use the estimated delay error for each channel to correct receive aberration in the ultrasound imaging data (225).

13. The processing system (210) of claim 11 or 12, wherein the weights and delays for the transmit beam are determined for each line of the ultrasound imaging data (225).

14. The processing system (210) of any of claims 11 to 13, wherein the weights and delays for the transmit beam are determined for each of a plurality of depths.

15. An ultrasound system (200), comprising:

the processing system (210) of any of claims 11 to 14; and
an ultrasound beamforming unit (220) configured to acquire ultrasound imaging data (225).

FIG. 1

FIG. 2

FIG. 3

400

FIG. 4

500

FIG. 5

FIG. 6

700

Receive ultrasound imaging data — 710

Estimate delay error — 720

Determine weights and delays — 730

Generate corrected ultrasound imaging data — 740

FIG. 7

Europäisches
Patentamt

European
Patent Office

Office européen
des brevets

# EUROPEAN SEARCH REPORT

**Application Number**

EP 24 18 2886

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/117393 A1 (KONINKLIJKE PHILIPS NV [NL]) 9 June 2022 (2022-06-09) | 1,3-11, 13-15 | INV. G01S7/52 |
| A | * abstract; figures 2, 3, 5, 6 * <br> * paragraphs [0001], [0022], [0023] * <br> * paragraphs [0037], [0039], [0050] * <br> * paragraphs [0051] - [0054], [0057] * <br> * paragraphs [0066], [0070], [0074] * <br> * paragraph [0079] - paragraph [0087] * | 2,12 | G01S15/89 <br> A61B8/08 |
| | ----- | | |
| X | US 2021/196238 A1 (ROBERT JEAN-LUC FRANCOIS-MARIE [US] ET AL) 1 July 2021 (2021-07-01) | 1,2,6-8, 10-15 | |
| A | * figures 2, 2a * <br> * paragraphs [0021], [0022], [0026] * <br> * paragraphs [0027], [0028], [0031] * <br> * paragraphs [0033], [0040], [0043] * | 3-5,9 | |
| | ----- | | |

**TECHNICAL FIELDS SEARCHED (IPC)**

G01S
A61B

The present search report has been drawn up for all claims

| Place of search | Date of completion of the search | Examiner |
|---|---|---|
| Munich | 18 November 2024 | Knoll, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 2886

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2024

| Patent document cited in search report | Publication date | Patent family member(s) | | Publication date |
|---|---|---|---|---|
| WO 2022117393 A1 | 09-06-2022 | CN | 116529629 A | 01-08-2023 |
| | | EP | 4256370 A1 | 11-10-2023 |
| | | JP | 2023552336 A | 15-12-2023 |
| | | US | 2023408662 A1 | 21-12-2023 |
| | | WO | 2022117393 A1 | 09-06-2022 |
| US 2021196238 A1 | 01-07-2021 | CN | 112119327 A | 22-12-2020 |
| | | EP | 3794370 A1 | 24-03-2021 |
| | | JP | 7378429 B2 | 13-11-2023 |
| | | JP | 2021523788 A | 09-09-2021 |
| | | US | 2021196238 A1 | 01-07-2021 |
| | | WO | 2019219485 A1 | 21-11-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5997479 A, Savord
- US 6013032 A, Savord
- US 6623432 B, Powers
- US 6283919 B, Roundhill
- US 6458083 B, Jago
- US 6443896 B, Detmer
- US 6530885 B, Entrekin
- US 5388461 A
- US 8137272 B2

### Non-patent literature cited in the description

- **KARAMAN et al.** A phase aberration correction method for ultrasound imaging. *IEEE Transactions on Ultrasonics, Ferroelectrics and Frequency Control*, 1993, vol. 40 (4), 275-282
- **ROBERT ; FINK**. Green's function estimation in speckle using the decomposition of the time reversal operator: Application to aberration correction in medical imaging. *J. Acoust. Soc. Am.*, 2008, vol. 123 (2), 866-877
- **MÅSØY et al.** Variance analysis of arrival time and amplitude estimates from random speckle signal. *J. Acoust. Soc. Am.*, 2007, vol. 121 (1), 286-297
- **MÅSØY et al.** Aberration correction in 2D echocardiography. *IEEE Transactions on Medical Imaging*, 2022
- **ALI et al.** Aberration correction in diagnostic ultrasound: A review of the prior field and current directions. *Zeitschrift für Medizinische Physik*, 2023, vol. 33 (3), 267-291